# EUROPEAN PATENT SPECIFICATION

(11) **EP 2 578 205 B1**
(45) Date of publication and mention of the grant of the patent: **24.10.2018**
(21) Application number: 11789420.4
(22) Date of filing: 26.05.2011
(51) Int. Cl.: A61K 8/9789, A61K 8/34, A61Q 13/00, A61Q 19/00, A23G 3/48, A61K 8/33, A61Q 5/02, A61Q 19/10, A61Q 19/08, A61K 31/045, A61K 31/085, A61L 9/013, A61L 9/16, A61K 36/14, A61K 36/185, A61K 36/534, A61K 36/537, A23L 33/10, A23L 33/105

(54) **SKIN TEMPERATURE ELEVATING AGENT, AND COSMETIC COMPOSITION, FOOD, AND SUNDRY ITEMS CONTAINING SAID SKIN TEMPERATURE ELEVATING AGENT**
MITTEL ZUR ERHÖHUNG DER HAUTTEMPERATUR SOWIE KOSMETISCHE ZUSAMMENSETZUNG, NAHRUNGSMITTEL UND VERSCHIEDENE ANDERE ARTIKEL MIT DEM MITTEL ZUR ERHÖHUNG DER HAUTTEMPERATUR
AGENT D'ÉLÉVATION DE LA TEMPÉRATURE DE LA PEAU, ET COMPOSITION COSMÉTIQUE, PRODUIT ALIMENTAIRE ET DIVERS OBJETS CONTENANT LEDIT AGENT D'ÉLÉVATION DE LA TEMPÉRATURE DE LA PEAU

(30) Priority: 31.05.2010 JP 2010125333
(43) Date of publication of application: 10.04.2013
(73) Proprietor: Shiseido Company, Ltd., Chuo-ku Tokyo 104-8010 (JP)
(72) Inventor: MORI, Keiko, Yokohama-shi, Kanagawa 224-8558 (JP); HAZE, Shinichiro, Yokohama-shi, Kanagawa 224-8558 (JP); GOZU, Yoko, Yokohama-shi, Kanagawa 224-8558 (JP); JOICHI, Atsushi, Yokohama-shi, Kanagawa 224-8558 (JP)
(74) Representative: Marro, Nicolas
(86) International application number: PCT/JP2011/002954
(87) International publication number: WO 2011/152006

(56) References cited:
- EP-A1- 1 914 678
- JP-A- 5 339 144
- JP-A- 9 047 480
- JP-A- 2001 048 740
- JP-A- 2002 226 415
- JP-A- 2002 226 416
- JP-A- 2004 131 436
- JP-A- 2007 503 827
- JP-A- 2008 100 977
- JP-A- 2009 139 371
- JP-A- 2010 503 744
- KR-A- 20090 076 671

## Description

### Technical Field

The present invention relates to a skin temperature elevating agent that raises the temperature of skin. The present invention also relates to a cosmetic composition, a food, and a sundry article containing the skin temperature elevating agent.

### Background Art

It is known in the modern society that the lack of exercise, lack of sleep, stress, etc., make an autonomic nerve unbalanced, thereby resulting in poor circulation of blood. When the blood circulation becomes poor, warm blood does not reach the periphery. Thus, the body temperature and skin temperature drop, causing the sensitivity to cold. Moreover, the supply of oxygen and nutrition from the blood is disrupted and waste products are accumulated, therefore slowing down the metabolism.

When the metabolism slows down, the recovery function of the skin from fatigue is lowered, resulting in the deterioration of aesthetic beauty due to bad complexion and the aging.

Hence, various attempts have been made in order to improve the symptoms associated with the poor blood circulation. For example, Patent Documents 1 and 2 disclose skin temperature elevating agents that improve the blood flow to raise the temperature of skin.

### Prior Art Documents

### Patent Documents

Patent Document 1: JP 2006-8575 A
Patent Document 2: JP 2005-68069 A

### Summary of the Invention

### Problem to be Solved

Since modern people are always subject to stressful conditions, there is a demand for the development of a further better skin temperature elevating agent.

The present invention has been made in view of the above-explained circumstances, and it is an object of the present invention to provide the cosmetic use of a skin temperature elevating agent that can raise the skin temperature more effectively.

### Solution to the Problem

According to the present invention, there is provided the cosmetic use of dihydro-β-ionol for skin temperature elevation.
In one embodiment, dihydro-β-ionol is provided as cosmetic composition.
In another embodiment, the cosmetic composition is a perfume, eau de toilette, eau de cologne, cream, milky lotion, skin lotion, massaging gel, massaging cream, foundation, face powder, lip stick, soap shampoo and rinse, body shampoo, body rinse, body powder, aerosol or bath additive.
This present disclosure also discloses a skin temperature elevating agent comprising one or more selected from a group consisting of: 4-methoxystyrene; an extract of clary sage; an extract of Japanese peppermint; an extract of juniper berry; dihydro-β-ionol; and geraniol.

According to the present disclosure, there is provided a skin temperature elevating agent comprising one or more selected from a group consisting of: 4-methoxystyrene; an extract of clary sage; an extract of Japanese peppermint; an extract of juniper berry; dihydro-β-ionol; and geraniol, at a total of equal to or greater than 0.001 mass% and equal to or lower than 50 mass%.

In addition, there is provided a cosmetic composition comprising the above-explained skin temperature elevating agent.

Furthermore, according to the present disclosure, there is provided a food containing the above-explained skin temperature elevating agent.

Moreover, according to the present disclosure, there is provided a sundry article containing the above-explained skin temperature elevating agent.

### Advantageous Effects of the Invention

The cosmetic use of dihydro-β-ionol according to the present invention can raise the skin temperature of cheeks since it comprises dihydro-β-ionol; optionally this can be used along with one or more selected from a group consisting of: an extract of Japanese peppermint; an extract of clary sage; and geraniol. Hence, the cosmetic use of dihydro-β-ionol according to the present invention can further effectively raise a skin temperature.

Moreover, the cosmetic composition, food, and sundry article according to the present disclosure contain the above-explained skin temperature elevating agent, and thus having a skin temperature elevating effect.

### Brief Description of the Drawings

FIG. 1 is a diagram illustrating test results; and
FIG. 2 is a diagram illustrating test results.

### Description of Embodiments

A skin temperature elevating agent according to the present disclosure contains at least an active constituent that is selected from following groups: 4-methoxystyrene; an extract of clary sage; an extract of Japanese peppermint; an extract of juniper berry; dihydro-β-ionol; and geraniol. The aromas of an extract of Japanese peppermint, an extract of clary sage, dihydro-β-ionol, and geraniol contained in this skin temperature elevating agent raise the skin temperature of cheeks when inhaled. Moreover, the aromas of an extract of juniper berry, an extract of Japanese peppermint, and 4-methoxystyrene contained in this skin temperature elevating agent raise the skin temperature of hand fingertips when inhaled. Accordingly, the skin temperature elevating agent according to the present disclosure is capable of raising the skin temperature more effectively.

Note that the term "skin temperature elevation" in the present disclosure means that a skin temperature become higher when smelling the aroma of the active constituent contained in the skin temperature elevating agent according to the present disclosure than a skin temperature when not smelling the aroma of the active constituent contained in the skin temperature elevating agent according to the present disclosure.

However, the skin temperature elevating agent according to the present disclosure exerts the skin temperature elevating effect not only when the aromas thereof are smelled but also when absorbed in the body through drinking and eating, and when absorbed through the skin.

A detailed explanation will be below given of skin temperature elevating agent according to the present disclosure.

### (Japanese Peppermint)

Japanese peppermint (scientific name: Mentha Arvensis var. piperascens) is known with a popular name of Japanese peppermint, and is a Japanese domestic herbaceous perennial of Labiatae Mentha. To obtain an extract of Japanese peppermint, the whole body of Japanese peppermint can be used directly or in a pulverized manner, but it is especially preferable to use the leaves thereof.

An example extract of Japanese peppermint is an extraction essence extracted and obtained directly from leaf portions, etc., or extracted and obtained from those cut in an appropriate size or pulverized, etc. after having been dried. A fraction produced by separation and refinement of the extract may be substituted therefor. The extracting method is not limited to any particular one, and typical essential oil extracting methods are applicable, such as distillation like a steam distillation method or a hot water distillation method, an organic solvent extraction method, and an oil-and-fat adsorptive extraction method, and in particular, the use of the steam distillation method is preferable. In general, peppermints are already well known as constituents that give a pleasant cooling sensation, but an effect of raising a skin temperature according to the present disclosure has been unknown at all.

### (Juniper Berry)

Juniper berry is a fruit of a Cupressaceae plant (scientific name: Juniperrus communis L.) originating from Europe. An example extract of juniper berry is an extraction essence extracted and obtained directly from the fruit of juniper berry, or extracted and obtained from such a fruit cut in an appropriate size or pulverized, etc., after having been dried. Moreover, it may be a fraction that can be obtained by separating and purifying the extraction essence. The extracting method is not limited to any particular one, and typical essential oil extracting methods are applicable, such as the distillation method like the steam distillation method or the hot water distillation method, the organic solvent extraction method, and the oil-and fat adsorptive extraction method, and in particular, the use of the steam distillation method is preferable. Regarding the juniper berry, a body fat combustion effect (see JP 2005-105188 A) and a precursor fat cell differentiation suppressing effect (see JP 2004-75640 A), etc., are already well known, but an effect of raising a skin temperature has been unknown at all.

### (Clary Sage)

Clary sage (scientific name: Salvia sclarea) is a biennial grass herb that is a kind of Labiatae Salvia. An example extract of clary sage is an extraction essence extracted and obtained directly from the spike at the time of blooming or the whole body, or extracted and obtained from those cut in an appropriate size or pulverized, etc. after having been dried. Moreover, it may be a fraction that can be obtained by separating and purifying the extraction essence. The extracting method is not limited to any particular one, and typical essential oil extracting methods are applicable, such as a distillation method like the steam distillation method or the hot water distillation method, the organic solvent extraction method, and the oil-and fat adsorptive extraction method, and in particular, the use of the steam distillation method is preferable. Regarding clary sage, an effect of increasing a testosterone concentration in body fluid (see JP 2006-137707 A), etc., is already well known, but an effect of raising a skin temperature according to the present disclosure has been unknown at all.

### (Dihydro-β-ionol)

Dihydro-β-ionol is an aroma constituent contained in the aroma of rose and yellow passion fruit, etc. Dihydro-β-ionol is contained in an extraction essence extracted and obtained from, for example, the blooms of roses. Moreover, a fraction thereof that can be obtained by separating and purifying the extraction essence may be used. The extracting method is not limited to any particular one, and typical essential oil extracting methods are applicable, such as a distillation method like the steam distillation method or the hot water distillation method, the organic solvent extraction method, and the oil-and-fat adsorptive extraction method, and in particular, the use of the steam distillation method is preferable. Since dihydro-β-ionol can be synthesized through a well known synthesis method, for example, a commercially available synthetic product may be used.

Dihydro-β-ionol can be expressed by following chemical formula 1.

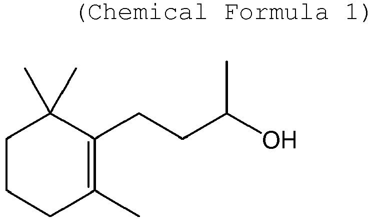

### (Geraniol)

Geraniol is an aroma constituent contained in many plant essential oils, such as palmarosa oil, geranium oil, and citronella oil, and can be obtained by separating and purifying those essential oils. Geraniol can be synthesized through a well known synthesis method, and for example, a commercially available synthesis product may be used.

Geraniol can be expressed by following chemical formula 2.

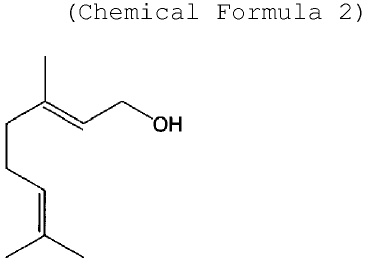

It is known that geraniol has a mental sedative effect (see JP 2003-119490 A) but an effect of raising a skin temperature according to the present disclosure has been unknown at all.

### (4-Methoxystyrene)

4-methoxystyrene is an aroma constituent contained in the aroma of rose, etc. 4-methoxystyrene is contained in an extraction essence extracted and obtained from the blooms of roses, etc. Moreover, a fraction obtained by separating and purifying the extraction essence may be used. The extracting method is not limited to any particular one, and typical essential oil extracting methods are applicable, such as a distillation method like the steam distillation method or the hot water distillation method, the organic solvent extraction method, and the oil-and-fat adsorptive extraction method, and in particular, the use of the steam distillation method is preferable. Since 4-methoxystyrene can be synthesized through a well known synthesis method, for example, a commercially available synthesis product may be used.

4-methoxystyrene can be expressed by following chemical formula 3.

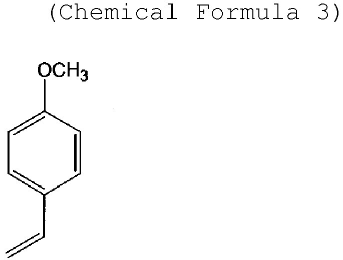

The skin temperature elevating agent cosmetically used according to the present invention is dihydro-β-ionol; optionally this can be used along with at least an active constituent that is selected from following groups: an extract of Japanese peppermint; an extract of juniper berry; an extract of clary sage; geraniol; and 4-methoxystyrene.

When the active constituent is volatilized from the skin temperature elevating agent, and a user inhales the aroma thereof, the above-explained skin temperature elevating effect is exerted. The skin temperature elevating agent may consist of the active constituents, but other arbitrary constituents, such as a dilution agent, an auxiliary agent, and an additive agent, may be contained as long as the amount thereof does not disturb the effect. When a desired additive agent, etc., is selected as needed and contained in the skin temperature elevating agent, such a skin temperature elevating agent can be utilized as a cosmetic composition, a medicinal drug, a quasi-drug, a food, a beverage, a sundry article, etc. That is, the skin temperature elevating agent can be a cosmetic composition, etc., having a skin temperature elevating effect.

On the other hand, when the skin temperature elevating agent is added to a predetermined composition like a cosmetic composition, the skin temperature elevating effect can be given to such a composition. That is, when the skin temperature elevating agent is added to a cosmetic composition, etc., a cosmetic composition, etc., having the skin temperature elevating effect can be obtained. Furthermore, when the skin temperature elevating agent is caused to permeate a cloth, a sundry article, etc., or added thereto, the above-explained active constituent volatilizes from the permeated or added skin temperature elevating agent, and thus the skin temperature elevating effect can be given to such a cloth, a sundry article, etc.

The containing amount of the active constituent in the skin temperature elevating agent is not limited to any particular one, but it is preferable that the containing amount should be equal to or greater than 0.0001 mass% and equal to or smaller than 100 mass%. It is more preferable that the containing amount should be equal to or greater than 0.001 mass% and equal to or smaller than 50 mass%, and it is further preferable that the containing amount should be equal to or greater than 0.01 mass% and equal to or smaller than 30 mass%.

When the skin temperature elevating agent is added to a composition, such as a cosmetic composition or a medicinal drug, to give the skin temperature elevating effect thereto, it is preferable that the containing amount of the active constituent in the skin temperature elevating agent to be added should be equal to or greater than 0.0001 mass% and equal to or smaller than 100 mass%. It is more preferable that such a containing amount should be equal to or greater than 0.001 mass% and equal to or smaller than 50 mass%, and it is further preferable that such a containing amount should be equal to or greater than 0.01 mass% and equal to or smaller than 30 mass%.

When the skin temperature elevating agent is added to a composition, such as a cosmetic composition or a medicinal drug, to give the skin temperature elevating effect thereto, it is preferable that the blending amount of the skin temperature elevating agent in the composition should be equal to or greater than 0.001 mass% and equal to or smaller than 100 mass%. It is especially preferable that such a blending amount should be equal to or greater than 0.003 mass% and equal to or smaller than 30 mass%.

The form of the skin temperature elevating agent according to the present invention is not limited to any particular one as long as it can exert the effect according to the present invention, and can be used in an arbitrary form, such as a liquid form, a paste form, a gel form, or a solid form.

Furthermore, the dosage form of the skin temperature elevating agent used according to the present invention is not limited to any particular one as long as it can exert the effect according to the present invention, and example dosage form thereof are a liquid form, a dust form, a granule form, an aerosol form, a solid form, and a gel form, but the present invention is not limited to those dosage forms.

There is no particular limitation for the cosmetic composition as one of especially suitable forms, but example cosmetic compositions are a fragrance such as perfume, eau du toilette, or au de cologne, cream, milky lotion, skin lotion, massaging gel, massaging cream, foundation, face powder, lip stick, soap, shampoo and rinse, body shampoo, body rinse, body powder, aerosol, bath additive, etc.

Moreover, when, for example, the skin temperature elevating agent according to the present disclosure is caused to permeate or is added to arbitrary sundry article, such as air freshener, air refresher, aroma candle, incense, stationery product, wallet, bag, and shoes, and arbitrary cloth, such as underwear, dress, hat, stocking, and socks, the above-explained active constituent volatilizes from the permeated or added skin temperature elevating agent, and thus the skin temperature elevating effect can be given to such a sundry article, a cloth, etc. The skin temperature elevating agent according to the present disclosure may be caused to permeate or added to the material of the sundry article, or cloth, etc., to produce a product from such a material, or the skin temperature elevating agent according to the present disclosure may be caused to permeate or added to the finished product. In addition, as other available human consumptions, the skin temperature elevating agent can be added to pill, tablet, candy, gum, etc.

Various use forms of the skin temperature elevating agent according to the present disclosure have been exemplified, but the present invention is not limited to those forms, and can be used in an arbitrary form as long as advantageous effects of the present invention can be exerted.

The present invention will be explained below in more detail with reference to examples.

Test samples were prepared which were a dilution of an extract of Japanese peppermint by triethyl citrate to a concentration of 0.05 mass%, a dilution of an extract of juniper berry by triethyl citrate to a concentration of 0.1 mass%, a dilution of an extract of clary sage by triethyl citrate to a concentration of 0.05 mass%, a dilution of dihydro-β-ionol by ethanol to a concentration of 0.1 mass%, a dilution of geraniol by ethanol to a concentration of 0.1 mass%, and a dilution of 4-methoxystyrene by ethanol to a concentration of 0.5 mass%, and how the aromas of those test samples influenced a skin temperature was tested.

An extract of Japanese peppermint available from NAGAOKA & Co., Ltd., an extract of juniper berry available from Charabot Corporation, an extract of clary sage available from Robertet Corporation, dihydro-β-ionol available from T. HASEGAWA Co., Ltd., geraniol available from IFF Japan Corporation, and 4-methoxystyrene available from TOKYO Chemical Industry, Co., Ltd., were prepared.

First of all, a test method applied for validation of the skin temperature elevating effect in the examples will be explained.

### (Test Method of Measuring Skin Temperature)

In this example, test subjects were females in their 20's. Five test subjects for the extract of Japanese peppermint, seven test subjects for the extract of juniper berry, three test subjects for the extract of clary sage, five test subjects for dihydro-β-ionol, five test subjects for geraniol, and seven test subjects for 4-methoxystyrene participated in the tests. The test content was explained in advance to the test subjects, and the participation in the test was agreed in writing.

The tests were carried out in a room at a constant temperature set to 25 °C and a constant humidity set to 45 %.

Skin temperature sensors were attached to a cheek of the test subject, a hand fingertip (middle finger), and a chest, and connected to a temperature monitor to successively measure the skin temperature. The temperature monitor used was N542R made by NIKKISO-Therm Co., Ltd. Skin temperature data was taken in a personal computer, and a skin temperature change level of a cheek and that of a hand fingertip were calculated with a chest skin temperature being as a reference temperature that had a skin temperature hardly changed. Skin temperature change levels of a cheek and those of a hand fingertip between when an aroma was smelled and when no aroma was smelled were compared with each other, and an effect of the aroma of the test sample to the skin temperature was evaluated.

When the test subjects were made to smell the aromas, the test samples were soaked cottons, and the cottons were brought below the noses of the test subjects to let the test subjects to smell the aromas through spontaneous breathing. Regarding the amounts of the test samples caused to soak into cottons, the extract of Japanese peppermint was 5 µl, the extract of juniper berry was 5 µl, the extract of clary sage was 5 µl, dihydro-β-ionol was 10 µl, geraniol was 5 µl, and 4-methoxystyrene was 5 µl. When the test subjects were caused to smell no aroma, cottons only were brought below the noses of the test subjects.

### (Test Result of Measuring Skin Temperature)

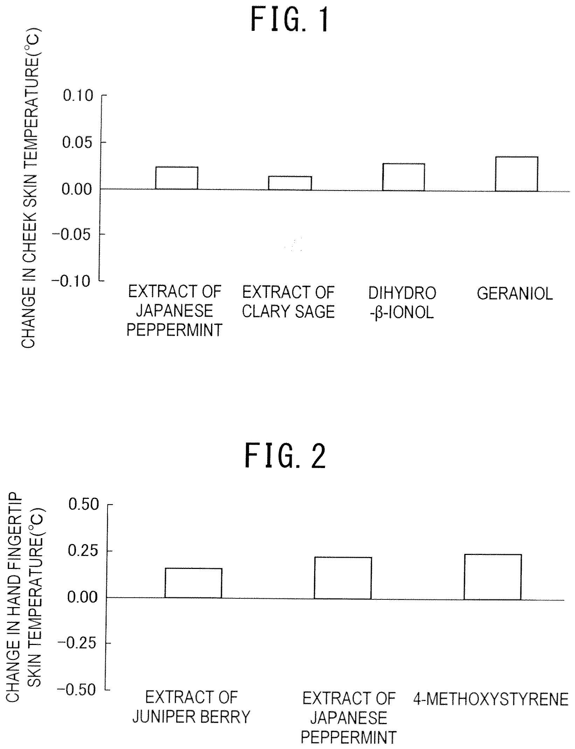

FIGS. 1 and 2 are diagrams illustrating test results.

Results are indicated by a value obtained by subtracting a skin temperature change level when no aroma was smelled from a skin temperature change level when the aroma was smelled. When this value is a positive one, it is determined that a skin temperature is raised and when this value is a negative one, it is determined that a skin temperature is lowered.

As is apparent from FIG. 1, the extract of Japanese peppermint, the extract of clary sage, dihydro-β-ionol, and geraniol have an effect of raising the skin temperature of the cheek of the test subject.

Moreover, as is apparent from FIG. 2, the extract of Japanese peppermint, the extract of juniper berry, and 4-methoxystyrene have an effect of raising the skin temperature of the hand fingertip of the test subject.

Hereinafter, examples of various cosmetic compositions, sundry articles and foods, and further fibers used for a cloth containing the skin temperature elevating agent will be explained in detail, but the present invention is not limited to those examples. As the skin temperature elevating agent according to the present disclosure, the extract of Japanese peppermint, the extract of clary sage, dihydro-β-ionol, geraniol, the extract of juniper berry, and 4-methoxystyrene were prepared solely or combined for blending. In the following, compositions comprising dihydro-β-ionol can be used in the cosmetic use according to the invention.

| Skin lotion | mass% |
|---|---|
| (1) Glycerin | 2.0 |
| (2) Dipropylene glycol | 2.0 |
| (3) Polyethylene-glycol-60-hydrogenated castor oil | 0.3 |
| (4) Xylitol | 3.0 |
| (5) Ascorbic acid | 0.005 |
| (6) Edetate trisodium | 0.1 |
| (7) Colorant | 0.1 |
| (8) Skin temperature elevating agent (extract of juniper berry) | 0.05 |
| (9) Purified water | balance |
| Total | 100 |

| Milky lotion | mass% |
|---|---|
| (1) Ethyl alcohol | 10.0 |
| (2) Glycerin | 3.0 |
| (3) Butylene glycol | 2.0 |
| (4) Polyethylene glycol | 3.0 |
| (5) Carboxyvinyl polymer | 0.1 |
| (6) Acrylic acid/alkyl acrylate copolymer | 0.1 |
| (7) Potassium hydroxide | 0.1 |
| (8) Cyclomethicone | 4.0 |
| (9) Squalane | 2.0 |
| (10) Spherical polyethylene | 2.0 |
| (11) Menthol | 0.5 |
| (12) Medicant | 1.0 |
| (13) Paraben | 0.1 |
| (14) Edetate trisodium | 0.1 |
| (15) Pigment | 0.1 |
| (16) Skin temperature elevating agent (extract of juniper berry) | 0.05 |
| (17) Purified water | balance |
| Total | 100 |

| Skin lotion | mass% |
|---|---|
| (1) Glycerin | 2.0 |
| (2) Dipropylene glycol | 2.0 |
| (3) Polyethylene-glycol-60-hydrogenated castor oil | 0.3 |
| (4) Xylitol | 3.0 |
| (5) Ascorbic acid | 0.005 |
| (6) Edetate trisodium | 0.1 |
| (7) Colorant | 0.1 |
| (8) Skin temperature elevating agent (extract of Japanese peppermint) | 0.05 |
| (9) Purified water | balance |
| Total | 100 |

| Milky lotion | mass% |
|---|---|
| (1) Ethyl alcohol | 10.0 |
| (2) Glycerin | 3.0 |
| (3) Butylene glycol | 2.0 |
| (4) Polyethylene glycol | 3.0 |
| (5) Carboxyvinyl polymer | 0.1 |
| (6) Acrylic acid/alkyl acrylate copolymer | 0.1 |
| (7) Potassium hydroxide | 0.1 |
| (8) Cyclomethicone | 4.0 |
| (9) Squalane | 2.0 |
| (10) Spherical polyethylene | 2.0 |
| (11) Menthol | 0.5 |
| (12) Medicant | 1.0 |
| (13) Paraben | 0.1 |
| (14) Edetate trisodium | 0.1 |
| (15) Pigment | 0.1 |
| (16) Skin temperature elevating agent (extract of Japanese peppermint) | 0.05 |
| (17) Purified water | balance |
| Total | 100 |

| Cream | mass% |
|---|---|
| (1) Glycerin | 10.0 |
| (2) Butylene glycol | 5.0 |
| (3) Carbomer | 0.1 |
| (4) Potassium hydroxide | 0.2 |
| (5) Stearic acid | 2.0 |
| (6) Glyceryl stearate | 2.0 |
| (7) Glyceryl isostearate | 2.0 |
| (8) Vaseline | 5.0 |
| (9) Preservative agent | 0.1 |
| (10) Antioxidant | 0.1 |
| (11) Skin temperature elevating agent (dihydro-β-ionol) | 0.3 |
| (12) Chelating agent | 1.0 |
| (13) Pigment | 0.01 |
| (14) Stearyl alcohol | 2.0 |
| (15) Behenyl alcohol | 2.0 |
| (16) Palm hardened oil | 2.0 |
| (17) Squalane | 10.0 |
| (18) 4-methoxy-potassium-salicylate | 3.0 |
| (19) Purified water | balance |
| Total | 100 |

| Cream | mass% |
|---|---|
| (1) Glycerin | 3.0 |
| (2) Dipropylene glycol | 7.0 |
| (3) Polyethylene glycol | 3.0 |
| (4) Glyceryl stearate | 3.0 |
| (5) Glyceryl isostearate | 2.0 |
| (6) Stearyl alcohol | 2.0 |
| (7) Behenyl alcohol | 2.0 |
| (8) Liquid paraffin | 7.0 |
| (9) Cyclomethicone | 3.0 |
| (10) Dimethicone | 1.0 |
| (11) Octyl-Methoxy-cinnamate | 0.1 |
| (12) Sodium hyaluronate | 0.05 |
| (13) Preservative agent | 0.1 |
| (14) Antioxidant | 0.1 |
| (15) Skin temperature elevating agent (geraniol) | 0.4 |
| (16) Chelating agent | 1.0 |
| (17) Pigment | 0.01 |
| (18) Purified water | balance |
| Total | 100 |

| Gel | mass% |
|---|---|
| (1) Ethyl alcohol | 10.0 |
| (2) Glycerin | 5.0 |
| (3) Butylene glycol | 5.0 |
| (4) Carbomer | 0.5 |
| (5) Aminomethyl-propanol | 0.3 |
| (6) Polyethylene-glycol-60-hydrogenated castor oil | 0.3 |
| (7) Menthol | 0.02 |
| (8) Preservative agent | 0.05 |
| (9) Chelating agent | 1.0 |
| (10) Skin temperature elevating agent (extract of Japanese peppermint) | 0.1 |
| (11) Purified water | balance |
| Total | 100 |

| Aerosol | mass% |
|---|---|
| (1) Glycerin | 2.0 |
| (2) Dipropylene glycol | 2.0 |
| (3) Polyethylene-glycol-60-hydrogenated castor oil | 0.3 |
| (4) Hydroxypropyl-p-cyclodextrin | 1.0 |
| (5) Preservative agent | 0.1 |
| (6) Chelating agent | 1.0 |
| (7) Colorant | 0.1 |
| (8) Skin temperature elevating agent (4-methoxystyrene) | 0.2 |
| (9) Purified water | 40.0 |
| (10) Liquid petroleum gas | balance |
| Total | 100 |

| Aerosol | mass% |
|---|---|
| (1) Alcohol | 15.0 |
| (2) Butylene glycol | 2.0 |
| (3) Glycerin | 1.0 |
| (4) Polypropylene-glycol-13-decyl-tetradec-24 | 0.1 |
| (5) Silver-supporting zeolite | 1.0 |
| (6) Chelating agent | 1.0 |
| (7) Colorant | 0.3 |
| (8) Skin temperature elevating agent (extract of juniper berry) | 0.15 |
| (9) Liquid petroleum gas | 40.0 |
| (10) Purified water | balance |
| Total | 100 |

| Aerosol | mass% |
|---|---|
| (1) Ethanol | 60.0 |
| (2) Menthyl lactate | 0.1 |
| (3) Sodium lactate | 0.1 |
| (4) Tocopherol acetate | 0.01 |
| (5) Lactic acid | 0.01 |
| (6) Caffeine | 0.01 |
| (7) Fennel essence | 1.0 |
| (8) Hamamelis essence | 1.0 |
| (9) Houttuynia cordata essence | 1.0 |
| (10) Dipropylene glycol | 1.0 |
| (11) Nitrogen gas | 0.9 |
| (12) Polyoxyethylene-polyoxypropylene-decyltetradecylether | 1.0 |
| (13) Butylene glycol | 2.0 |
| (14) Tocopherol | 0.05 |
| (15) Skin temperature elevating agent (extract of Japanese peppermint) | 0.1 |
| (16) Polyethylene-glycol-60-hydrogenated castor oil | 0.1 |
| (17) Purified water | balance |
| Total | 100 |

| Shampoo | mass% |
|---|---|
| (1) Lauryl-polyoxyethylene (3) sulfate ester sodium salt (30 % aqueous solution) | 30.0 |
| (2) Lauryl-sulfate ester sodium salt (30 % aqueous solution) | 10.0 |
| (3) Palm oil fatty acid diethanolamide | 4.0 |
| (4) Glycerin | 1.0 |
| (5) Preservative agent | 0.1 |
| (6) Skin temperature elevating agent (extract of juniper berry) | 0.5 |
| (7) Dye | 0.1 |
| (8) Sequestering agent | 0.1 |
| (9) pH adjuster | 0.5 |
| (10) Purified water | balance |
| Total | 100 |

| Rinse | mass% |
|---|---|
| (1) Silicone oil | 3.0 |
| (2) Liquid paraffin | 1.0 |
| (3) Cetyl alcohol | 1.5 |
| (4) Stearyl alcohol | 1.0 |
| (5) Stearyl-chloride-trimethyl-ammonium | 0.7 |
| (6) Glycerin | 3.0 |
| (7) Skin temperature elevating agent (extract of clary sage) | 0.5 |
| (8) Dye | 0.1 |
| (9) Preservative agent | 0.05 |
| (10) Purified water | balance |
| Total | 100 |

| Body shampoo | mass% |
|---|---|
| (1) Lauric acid | 2.5 |
| (2) Myristic acid | 5.0 |
| (3) Palmitic acid | 2.5 |
| (4) Oleic acid | 2.5 |
| (5) Cocoyl diethanol amide | 1.0 |
| (6) Glycerin | 20.0 |
| (7) Potassium hydroxide | 3.6 |
| (8) Colorant | 0.1 |
| (9) Skin temperature elevating agent (4-methoxystyrene) | 0.5 |
| (10) Sequestering agent | 0.1 |
| (11) Purified water | balance |
| Total | 100 |

| Fragrance | mass% |
|---|---|
| (1) Alcohol | 75.0 |
| (2) Dipropylene glycol | 5.0 |
| (3) Skin temperature elevating agent (extract of juniper berry) | 10.0 |
| (4) Antioxidant | 8.0 |
| (5) Dye | 0.01 |
| (6) Ultraviolet absorber | 0.01 |
| (7) Purified water | balance |
| Total | 100 |

| Room fragrance | mass% |
|---|---|
| (1) Alcohol | 80.0 |
| (2) Antioxidant | 5.0 |
| (3) Skin temperature elevating agent (extract of clary sage) | 3.0 |
| (4) 3-methyl-3-methoxybutanol | 5.0 |
| (5) Dibenzylidene sorbitol | 5.0 |
| (6) Purified water | balance |
| Total | 100 |

| Incense | mass% |
|---|---|
| (1) Thunbergii powder | 75.5 |
| (2) Sodium benzoate | 15.5 |
| (3) Skin temperature elevating agent (extract of juniper berry) | 5.0 |
| (4) Eucalyptus oil | 1.0 |
| (5) Fennel oil | 1.0 |
| (6) Purified water | balance |
| Total | 100 |

| Bath additive | mass% |
|---|---|
| (1) Sodium sulfate | 45.0 |
| (2) Sodium hydrogen carbonate | 45.0 |
| (3) Hyssop oil | 9.0 |
| (4) Skin temperature elevating agent (geraniol) | 1.0 |
| Total | 100 |

| Massage gel | mass% |
|---|---|
| (1) Erythritol | 2.0 |
| (2) Caffeine | 5.0 |
| (3) Phellodendron corktree extract | 3.0 |
| (4) Glycerin | 50.0 |
| (5) Carboxyvinyl polymer | 0.4 |
| (6) Polyethylene glycol 400 | 30.0 |
| (7) Edetate trisodium | 0.1 |
| (8) Polyoxirene (10) methyl polysiloxane copolymer | 2.0 |
| (9) Squalane | 1.0 |
| (10) Potassium hydroxide | 0.15 |
| (11) Skin temperature elevating agent (extract of juniper berry) | 1.0 |
| (12) Purified water | balance |
| Total | 100 |

| Massaging cream | mass% |
|---|---|
| (1) Solid paraffin | 5.0 |
| (2) Beeswax | 10.0 |
| (3) Vaseline | 15.0 |
| (4) Liquid paraffin | 41.0 |
| (5) 1,3-butylene glycol | 4.0 |
| (6) Monostearic acid glycerin | 2.0 |
| (7) Polyoxirene (20) sorbitan monolaurate | 2.0 |
| (8) Borax | 0.2 |
| (9) Caffeine | 2.0 |
| (10) Preservative agent | 0.1 |
| (11) Antioxidant | 0.1 |
| (12) Skin temperature elevating agent (extract of clary sage) | 1.0 |
| (13) Purified water | balance |
| Total | 100 |

### Aromatic Fiber

A microcapsule (particle size: equal to or smaller than 50 µm, and the ratio of the skin temperature elevating agent relative to the micro capsule was 50 mass%) containing the skin temperature elevating agent (e.g., an extract of juniper berry) was added in and mixed with a cuproammonium cellulose solution (cellulose concentration: 10 mass%, ammonium concentration: 7 mass%, and copper concentration: 3.6 mass%) within a range of 0.1 to 20 mass% relative to the cellulose mass, then fiber spinning was performed in accordance with a normal wet fiber spinning technique, and aromatic fibers were obtained through a refining process and a drying process .

| Granulated powder | mass% |
|---|---|
| (1) Sucralose | 0.1 |
| (2) Skin temperature elevating agent (extract of juniper berry) | 0.1 |
| (3) Skin temperature elevating agent (extract of clary sage) | 0.1 |
| (4) Flavor | 5.0 |
| (5) Diluting agent (ceolus) | 10.0 |
| (6) Maltitol | balance |
| Total | 100 |

| Pill (chewable type) | mass% |
|---|---|
| (1) Inositol | 11.0 |
| (2) Maltitol | 21.0 |
| (3) Sucrose | 0.5 |
| (4) Salmon milt extract (DNA Na) | 0.1 |
| (5) Yeast extract | 0.1 |
| (6) Skin temperature elevating agent (extract of juniper berry) | 0.1 |
| (7) Flavor | 5.0 |
| (8) Diluting agent | balance |
| Total | 100 |

| Tablet | mass% |
|---|---|
| (1) Lubricant (sucrose fatty acid ester, etc.) | 1.0 |
| (2) Gum arabic solution (5 %) | 2.0 |
| (3) Acidulant | 1.0 |
| (4) Artificial color | 0.01 |
| (5) Skin temperature elevating agent (extract of Japanese peppermint) | 0.1 |
| (6) Carbohydrate (powder sugar or sorbitol, etc.) | balance |
| Total | 100 |

| Candy | mass% |
|---|---|
| (1) Sugar | 50.0 |
| (2) Starch syrup | 47.95 |
| (3) Organic acid | 2.0 |
| (4) Skin temperature elevating agent (extract of juniper berry) | 0.05 |
| Total | 100 |

| Gum | mass% |
|---|---|
| (1) Sugar | 43.0 |
| (2) Gum base | 30.95 |
| (3) Glucose | 10.0 |
| (4) Starch syrup | 16.0 |
| (5) Skin temperature elevating agent (extract of clary sage) | 0.05 |
| Total | 100 |

## Claims

1. Cosmetic use of dihydro-β-ionol for skin temperature elevation.

2. Cosmetic use of dihydro-β-ionol according to claim 1, wherein dihydro-β-ionol is provided as cosmetic composition.

3. Cosmetic use of dihydro-β-ionol according to claim 2, wherein the cosmetic composition is a perfume, eau de toilette, eau de cologne, cream, milky lotion, skin lotion, massaging gel, massaging cream, foundation, face powder, lip stick, soap shampoo and rinse, body shampoo, body rinse, body powder, aerosol or bath additive.

## Patentansprüche

1. Kosmetische Verwendung von Dihydro-β-Ionol zur Erhöhung der Hauttemperatur.

2. Kosmetische Verwendung von Dihydro-β-Ionol nach Anspruch 1, wobei Dihydro-β-Ionol als kosmetische Zusammensetzung bereitgestellt wird.

3. Kosmetische Verwendung von Dihydro-β-Ionol nach Anspruch 2, wobei die kosmetische Zusammensetzung ein/e Parfum, Eau de Toilette, Eau de Cologne, Creme, milchige Lotion, Hautlotion, Massagegel, Massagecreme, Grundierung, Gesichtspuder, Lippenstift, Seifenshampoo und -spülung, Körpershampoo, Körperspülung, Körperpuder, Aerosol oder Badezusatz ist.

## Revendications

1. Utilisation cosmétique de dihydro-β-ionol pour une élévation de la température de la peau.

2. Utilisation cosmétique de dihydro-β-iοnol selon la revendication 1, dans laquelle le dihydro-β-iοnol est fourni comme une composition cosmétique.

3. Utilisation cosmétique de dihydro-β-ionol selon la revendication 2, dans laquelle la composition cosmétique est un parfum, une eau de toilette, une eau de Cologne, une crème, une lotion de lait, une lotion pour la peau, un gel de massage, une crème de massage, un fond de teint, une poudre pour le visage, un stick à lèvres, un shampoing au savon et rinçage, un shampoing pour le corps, un rinçage pour le corps, une poudre pour le corps, un aérosol ou un additif pour le bain.
